# EUROPEAN PATENT SPECIFICATION

(11) **EP 3 047 872 B1**
(45) Date of publication and mention of the grant of the patent: **04.10.2017**
(21) Application number: 16152298.2
(22) Date of filing: 21.01.2016
(51) Int. Cl.: A61M 39/10, A61M 39/20

(54) **VALVED MALE LUER CONNECTOR**
MÄNNLICHER LUER-STECKER MIT VENTIL
CONNECTEUR MÂLE LUER À VALVE

(30) Priority: 21.01.2015 IT TO20150045
(43) Date of publication of application: 27.07.2016
(73) Proprietor: Industrie Borla S.p.A., 10024 Moncalieri (Torino) (IT)
(72) Inventor: GUALA, Gianni, I-10133 Torino (IT)
(74) Representative: Buzzi, Franco

(56) References cited:
- EP-A2- 2 504 056
- US-A1- 2003 032 940
- US-A1- 2006 192 164

## Description

### Field of the invention

The present invention generally refers to medical fluid connectors, and more in particular it regards a valved male luer connector applicable to a fluid line and adapted to be connected to a connector generally of the female type and the like for opening the flow passage through the two connectors.

### State of the prior art

A valved male luer connector comprising a casing, a tubular member having an inlet portion configured for connection to a medical line and an outlet portion with an open terminal end is known from European patent n° EP-2504056B1 in the name of the Applicant. An elastic hollow element anchored to the casing surrounds the tubular member and it has a terminal wall normally sealingly closing the terminal end of the outlet portion of the tubular member, and a cut. A collar is interposed between the casing and the elastic hollow element and it is axially displaceable, following the thrust engagement by a female connector that can be connected to the male connector, to cause a stretching deformation of the elastic hollow element with the consequent opening of the cut and thus the flow passage between the inlet portion and outlet portion of the tubular member. US2006/0192164 and US2003/3032940 are disclosing analogous valved male luer connectors.

### Summary of the invention

The present invention regards an improvement of the aforementioned known valved male luer connector, in particular as regards an easier and safer connection of the connector to the medical line.

According to the invention this object is attained by the features set forth in claim 1.

Thanks to the characterising feature of claim 1, when the valved male luer connector is connected, in use, to the medical line, typically constituted by a flexible duct, it can be manipulated so as to be connected to the female connector not only in an easier fashion, due to the fact that any twisting of the medical line is efficiently avoided, but also more safely since it avoids the risk of inadvertent detachment of the medical line due to a torsion thereof.

The tubular member is freely rotatable with respect to the casing in both opposite directions of rotation, and according to a preferred embodiment of the invention, the tubular member of the connector is made in a single piece with the inlet portion thereof and it is rotatable as a whole with respect to the casing. In this case, the body of the connector has, on the side of such inlet portion, a hollow appendage through which the tubular member is rotatably engaged following an axial snap-fit coupling thereof during the assembly of the connector.

### Brief description of the drawings

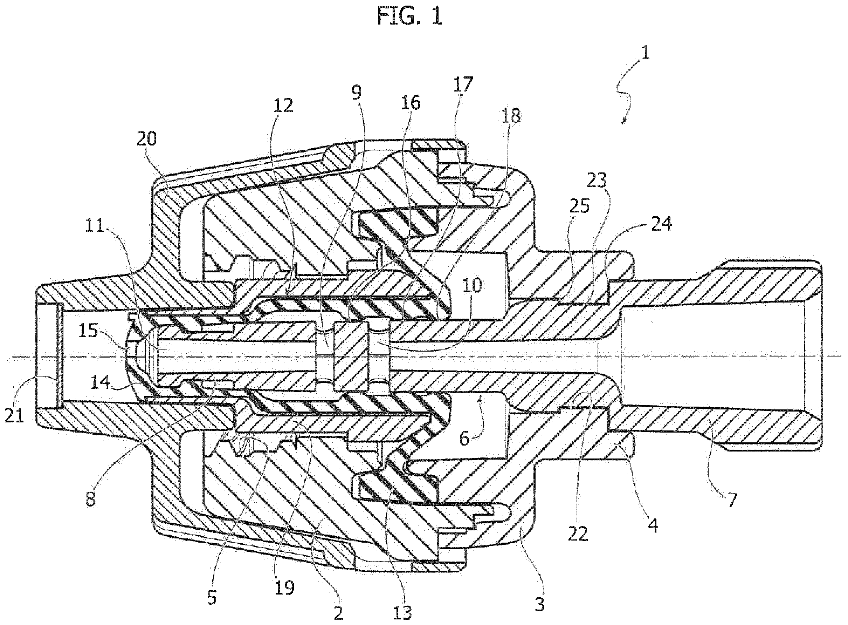

The invention will now be described in detail with reference to Figure 1, provided purely by way of nonlimiting example, representing an axial sectional view of the valved connector according to the invention.

### Detailed description of the invention

With reference to the figure, the valved male luer connector according to the invention comprises (as generally known from the aforementioned document EP-2504056B1) a casing 1 constituted by a hollow body 2 and an annular flange part 3 permanently axially coupled to the hollow body 2 and formed, on the opposite side, with a tubular appendage 4 to be described hereinafter.

The hollow body 2 has, on the side opposite to the flange 3, an internally threaded seat 5.

A tubular member which is axially extended through the body 1 is indicated in its entirety with 6 and it is rotatable with respect thereto through the methods described hereinafter.

The tubular member 6 has an inlet portion 7, preferably formed integrally joined and projecting to the external of the tubular appendage 4, configured for coupling a flexible duct of a medical line.

On the opposite side, the tubular member 6 has an outlet portion 8 which projects to the external of the threaded seat 5 to define a male luer lock connector adapted to be engaged by a supplementary female connector, not illustrated in that of the per se known type. For example, the female connector may be of the valved type described and illustrated in document EP-834665B1, also on behalf of the Applicant.

The inlet 7 and outlet 8 portions are connected to each other through a series of spaced radial passages 9, 10 whose mutual communication is controlled as clarified hereinafter, and the outlet portion 8 has an open terminal end indicated with 11.

Indicated with 12 is an elastic hollow element, typically made of elastomeric or silicone rubber, including an annular base 13 anchored in the casing 1 between the body 2 and the flange 3, and a terminal transversal wall 14 normally arranged facing the terminal end 11 of the tubular member 6 and having a cut 15 normally closed so as to substantially sealingly occlude the terminal end 11.

The elastic hollow element 12 internally has a first, a second and a third sealing annular relief respectively indicated with 16, 17 and 18 at contact with the tubular member 6. The annular relief 16 interacts with the passages 9 and 10 for respectively closing and opening the communication between the inlet portion 7 and the outlet portion 8 of the tubular member 6: in the condition represented in the figure, the passages 9 and 10 are insulated with respect to each other.

A collar interposed between the body 2 of the casing 1 and the elastic hollow element 12 and constituting an actuator member adapted to control the opening of the flow passage through the connector due to a stretching deformation of the elastic hollow element 12 is indicated with 19. The collar 19 is predisposed to cooperate, as described in the previously mentioned document EP-2504056B1, with a female luer connector or the like coupled, in use, with the seat 5 of the body 2. Due to such coupling the female connector exerts an axial thrust against the collar 19 so as to push it in the direction of the inlet portion 7 and elastically stretch deforming the elastic hollow element 12, so as to open the flow passage through the cut 15.

A priming cap which can be removably applied to the casing 1 to keep the connector in a condition of at least partial opening before the coupling thereof with the female connector is indicated with 20. Thus, in use, the medical liquid coming from the duct connected to the tubular appendage 7 is capable of reaching the terminal end 11 of the tubular member 6 without requiring a priming intervention by the operator, thus making the valved male luer connector ready for coupling thereof to the female connector and allow the supply of the medical liquid to a patient. The priming cap 20, which is provided with a liquid-impermeable transversal barrier 21, is rotatably displaceable between a first axial position in which the valved connector remains closed, and a second axial position in which it interacts with the collar 19 so as to stretch deform the elastic element 12 freeing the communication between the radial passages 9 and 10 and thus between the inlet 7 and outlet 8 portions of the tubular member 6, simultaneously opening the cut 15. Thus, the inlet end 7 and the duct, in the use related thereto, are kept in communication with the atmosphere through the membrane 21 which, as mentioned, constitutes a barrier impermeable to the liquid coming from the duct. Thus, the connector according to the invention is ready for the subsequent coupling with the female connector without requiring further priming operations: as a matter of fact, it is sufficient to remove the cap 20 to be able to apply the female connector.

As mentioned previously, the tubular member 6 with the relative inlet portion 7 is, according to the distinctive characteristic of the invention, freely rotatable with respect to the casing 1 in both opposite directions of rotation. For this purpose, the tubular appendage 4 of the flange 3 is formed with an inner annular relief 22 with which there is engaged an annular groove 22 of the tubular member 6, formed between two axial stop steps 24, 25 of which the second, the innermost one, has a smaller diameter. Thus, during the assembly of the connector the tubular member 6 is axially inserted into the casing 1 up to obtaining a snap-fit coupling of the annular groove 23 with respect to the annular relief 22 of the flange 3. Following such snap-fit coupling, the tubular member 6 with the inlet portion 7 is rotatable and axially locked with respect to the casing 1. Thus, in use, the casing 1 is capable of rotating with respect to the duct of the medical line fixed to the inlet portion 7, thus being advantageous not only for an easier handling of the connector, but also as regards the safety thereof: The free rotation between the connector and the duct actually prevents the latter from twisting and possibly inadvertently separating from the connector.

Obviously, the construction details and the embodiments may widely vary with respect to what has been described and illustrated, without departing from the scope of protection of the present invention as defined in the claims that follow. Thus, for example, though in the described embodiment the inlet portion 7 or 7a is made of a single piece with the tubular member 6, it could also be separated and thus rotatable independently from such tubular member.

## Claims

1. Valved male luer connector comprising a casing (1), a tubular member (6) having an inlet portion (7) configured for connection to a medical line and an outlet portion (8) with a terminal open end (11), an elastic hollow element (12) secured to the housing (1) and enclosing the tubular member (6), said elastic hollow element (12) having a terminal wall (14) normally sealingly closing said terminal end (11) of the outlet portion (8) of the tubular member (6) and having a cut (15), and a collar (19) interposed between the casing (1) and the elastic hollow element (12) and axially displaceable, following thrust engagement by a female connector connectable to said male connector, to cause a stretching deformation of said elastic hollow element (12) with the consequent opening of flow passage between said inlet portion (7) and said outlet portion (8) of the tubular member (6), **characterized in that** said inlet portion (7) is axially locked and freely rotatable with respect to the casing (1) in both opposite directions of rotation.

2. Connector according to claim 1, **characterized in that** said tubular member (6) as a whole is rotatable with respect to the casing (1).

3. Connector according to claim 2, **characterized in that** the casing (1) is provided, on the side of said outlet portion (8), with a hollow appendage (4) through which said tubular member (6) is rotatably engaged following an axial snap-fit coupling (22, 23) thereof.

4. Connector according to any one of the preceding claims, **characterized in that** it further includes a priming cap (20) designed to be releasably connected to the casing (1) to keep the outlet portion (8) of the tubular member (6) in communication with the atmosphere through a liquid-impermeable barrier (21).

## Patentansprüche

1. Männlicher Luer-Stecker mit Ventil, umfassend ein Gehäuse (1), ein röhrenförmiges Glied (6) mit einem zur Verbindung mit einer medizinischen Leitung konfigurierten Einlassteil (7) und einem Auslassteil (8) mit einem abschließenden offenen Ende (11), ein an dem Gehäuse (1) befestigtes und das röhrenförmige Glied (6) umschließendes elastisches hohles Element (12), wobei das elastische hohle Element (12) eine abschließende Wand (14) aufweist, die normalerweise das abschließende Ende (11) des Auslassteils (8) des röhrenförmigen Glieds (6) dicht verschließt und einen Schnitt (15) aufweist, und eine Manschette (19), die zwischen dem Gehäuse (1) und dem elastischen hohlen Element (12) eingefügt und nach Schubeingriff durch einen mit dem männlichen Stecker verbindbaren weiblichen Stecker axial verschiebbar ist, um eine dehnende Verformung des elastischen hohlen Elements (12) mit daraus folgender Öffnung des Durchflusskanals zwischen dem Einlassteil (7) und dem Auslassteil (8) des röhrenförmigen Glieds (6) zu bewirken, **dadurch gekennzeichnet, dass** der Einlassteil (7) axial verriegelt und bezüglich des Gehäuses (1) in beide entgegengesetzte Drehrichtungen frei drehbar ist.

2. Stecker nach Anspruch 1, **dadurch gekennzeichnet, dass** das röhrenförmige Glied (6) als Ganzes bezüglich des Gehäuses (1) drehbar ist.

3. Stecker nach Anspruch 2, **dadurch gekennzeichnet, dass** das Gehäuse (1) auf der Seite des Auslassteils (8) mit einem hohlen Ansatz (4) versehen ist, durch den das röhrenförmige Glied (6) nach einer axialen Schnappkupplung (22, 23) desselben drehbar in Eingriff ist.

4. Stecker nach einem der vorstehenden Ansprüche, **dadurch gekennzeichnet, dass** er ferner eine Ansaugkappe (20) umfasst, die dazu bestimmt ist, lösbar mit dem Gehäuse (1) verbunden zu werden, um den Auslassteil (8) des röhrenförmigen Glieds (6) durch eine flüssigkeitsundurchlässige Barriere (21) in Kommunikation mit der Atmosphäre zu halten.

## Revendications

1. Connecteur Luer mâle à valve comprenant un boîtier (1), un élément tubulaire (6) ayant une partie d'entrée (7) configurée pour se raccorder à une ligne médicale et une partie de sortie (8) avec une extrémité terminale ouverte (11), un élément creux élastique (12) fixé sur le boîtier (1) et enfermant l'élément tubulaire (6), ledit élément creux élastique (12) ayant une paroi terminale (14) fermant normalement de manière étanche ladite extrémité terminale (11) de la partie de sortie (8) de l'élément tubulaire (6) et ayant une découpe (15), et un collier (19) intercalé entre le boîtier (1) et l'élément creux élastique (12) et axialement déplaçable, suite à la mise en prise de poussée par un connecteur femelle pouvant être raccordé audit connecteur mâle, afin de provoquer une déformation d'étirement dudit élément creux élastique (12) avec l'ouverture conséquente du passage d'écoulement entre ladite partie d'entrée (7) et ladite partie de sortie (8) de l'élément tubulaire (6), **caractérisé en ce que** ladite partie d'entrée (7) est axialement verrouillée et librement rotative par rapport au boîtier (1) dans les deux directions de rotation opposées.

2. Connecteur selon la revendication 1, **caractérisé en ce que** ledit élément tubulaire (6), dans son ensemble, peut tourner par rapport au boîtier (1).

3. Connecteur selon la revendication 2, **caractérisé en ce que** le boîtier (1) est prévu, du côté de ladite partie de sortie (8), avec un appendice creux (4) à travers lequel ledit élément tubulaire (6) est mis en prise, en rotation, suite à son couplage par encliquetage axial (22, 23).

4. Connecteur selon l'une quelconque des revendications précédentes, **caractérisé en ce qu'**il comprend en outre un capuchon d'amorçage (20) conçu pour être raccordé, de manière amovible, au boîtier (1) afin de maintenir la partie de sortie (8) de l'élément tubulaire (6) en communication avec l'atmosphère par le biais d'une barrière imperméable au liquide (21).
